# EUROPEAN PATENT APPLICATION

(11) **EP 4 541 363 A1**
(43) Date of publication of application: **23.04.2025**
(21) Application number: 23819306.4
(22) Date of filing: 01.06.2023
(51) Int. Cl.: A61K 36/02, A61K 31/025, A61K 31/045, A61P 33/04

(54) **USE OF THE (+)-ELATOL COMPOUND FOR TREATING OR PREVENTING A NAEGLERIA FOWLERI INFECTION**

(30) Priority: 06.06.2022 ES 202230496
(71) Applicant: Universidad de la Laguna, 38508 La Laguna (ES)
(72) Inventor: FERNÁNDEZ CASTRO, José Javier, 38200 LA LAGUNA (ES); PEIXOTO NOCCHI CARNEIRO, Nathalia, 38200 La Laguna (ES); DÍAZ MARRERO, Ana Raquel, 38200 La Laguna (ES); LORENZO MORALES, Jacob, 38200 La Laguna (ES); PIÑERO BARROSO, José, 38200 La Laguna (ES); ARBERAS JIMÉNEZ, Íñigo, 38200 La Laguna (ES); SIFAOUI, Inés, 38200 La Laguna (ES); RIZO LIENDO, Aitor, 38200 La Laguna (ES)
(74) Representative: Isern Patentes y Marcas S.L.
(86) International application number: PCT/ES2023/070364
(87) International publication number: WO 2023/237795

(57) **Abstract**

The present invention relates to the use of an elatol compound for treating or preventing primary amebic meningoencephalitis (PAM) caused by Naegleria fowleri in a human or animal subject. The invention also relates to the pharmaceutical composition and kit that comprise said compound.

## Description

### Technical sector

The present invention belongs to the field of medicine and microbiology. It relates to active ingredients against the ameba *Naegleria fowleri* and to the treatment and/or prophylaxis of the infection and the Primary Amebic Meningoencephalitis (PAM) disease it causes.

### Background of the invention

*Naegleria* is a protist genus of free-living amoebas belonging to the Vahlkampfiidae family and the Heterolobosea class. It is commonly found in warm freshwater between 25 and 46°C in lakes, rivers, hot springs, industrial water sources or domestic water supplies, and in soils worldwide.

Application RU2649091 discloses a method for combating the proliferation of *Naegleria fowleri* in a medium by using another biological agent, *Willaertia magna.*

*Naegleria fowleri,* also known as "brain-eating ameba", is the only species in the genus that is pathogenic to humans. The microbe enters the brain and causes a disease of the central nervous system called Primary Amebic Meningoencephalitis (PAM). It is an acute infection that is very difficult to detect and causes cerebral edema, hemorrhage, necrosis and a massive and fulminant inflammatory response of brain tissue, which ultimately results in a coma and rapid death of the patient.

Management of PAM due to *N. fowleri* is the main challenge for all countries with prevalence of the disease. However, there is currently no adequate effective treatment to ensure the patient's survival.

Most information on the therapeutic efficacy of drugs against *N. fowleri* is based on case reports or *in vitro* studies, with varying therapeutic success. The effectiveness of the treatments is unclear, as almost all infections have been fatal and successful results are controversial. The treatment of the disease is largely influenced by the actuation time, which is extremely limited and not well defined; currently it is only possible through a strategy called drug "repurposing" with a combined therapeutic cocktail that includes known antifungals, antibiotics and microbicides.

The antifungal drug amphotericin B has been the mainstay of treatment of PAM (Gharpure R. et al. "Epidemiology and Clinical Characteristics of Primary Amebic meningoencephalitis Caused by Naegleria fowleri: A Global Review"; Clinical Infectious Diseases, 2021, Vol. 73, Issue 1). It is used in all infected patients without really having universal efficacy, as only 8% of patients recover at least partially after its administration either alone or in combination with other medicines, which makes it difficult to determine its effectiveness. The major drawback of amphotericin B is its high toxicity, which greatly influences its administration and the evolution of the patient.

Application GB2592782 A describes miltefosine for treating free-living amebic infections, including *N. fowleri.* The authors confirm the survival of three patients worldwide after treatment with miltefosine. In fact, the results in combined therapy are promising and successful, although mortality rates remain very high even with the best regimens available.

WO2022/008761 discloses synthetic heterocyclic compounds exhibiting some activity against *Naegleria fowleri,* and patent US10377733B1 finds activity in compounds with a sesquiterpene structure, in particular.

The use and study of natural substances, especially plants and micro-organisms, has been very relevant for treating diseases caused by protozoan parasites. Recently, some work has evaluated the potential of compounds extracted from algae compared to amoebas. In particular, laurane and cyclolaurane sesquiterpenes isolated from *Laurencia johnstonii* have shown good activity for *Naegleria fowleri* (Arberas-Jiménez I. et al. "Laurinterol from Laurencia johnstonii eliminates Naegleria fowleri triggering PCD by inhibition of ATPases"; Scientific Reports, 10, 17731, 2020). However, their Selectivity Indexes (SI) do not meet the established criteria to be considered as dewormers suitable for their administration.

Unfortunately, the medical treatments currently administered to combat PAM have little therapeutic efficacy and multiple serious side effects, which are often irreversible in those patients who survive. The major drawback of all treatments used today to treat a *Naegleria fowleri* infection is their high toxicity and low effectiveness, for which reason their clinical use is very limited.

Sesquiterpenes from marine origin are a family of compounds of a wide variability that can be isolated from algae of the genus *Laurencia* and invertebrates. They currently constitute more than 575 structures (Cikoš, A. M. *et al.* "Update on sesquiterpenes from red macroalgae of the *Laurencia* genus and their biological activities (2015-2020)"; Algal Research, 56, 102330, 2021).

Among them, chamigrene sesquiterpenes are highly representative and account for about 30% of all isolated sesquiterpenes. They are characterized by a spiro[5.5]undecane skeleton in which a stereogenic quaternary carbon (C-6) links the spirocycles, with a high variability of substitution with functional groups.

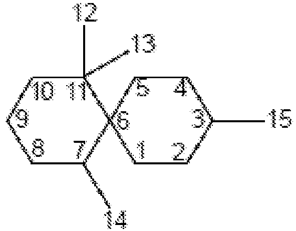

The α-chamigrenes, β-chamigrenes and nor-chamigrenes are not functionally equivalent to each other, apart from the fact that other types of functionalization have also been described (Harizani, M. et al. "The Laurencia paradox: An endless source of chemodiversity"; Progress in the Chemistry of Organic Natural Products 102, 91-252, 2016).

The chamigrene family has a broad biological spectrum, with antibacterial, antifungal, antiviral, anti-inflammatory, cytotoxic and antiparasitic activity. Specific antiparasitic activity would be exhibited by the β-chamigrenes, nor-chamigrenes and chamigrenes with oxygenation capacity at C-7, chamigrenes featuring a 5,10-epoxide ring or a carbocycle with prior migration of a methyl from C-11 to C-10.

For many of these compounds, partial or total synthesis has already been successfully achieved. In the same manner, natural chamigrene skeletons serve as a model for the synthesis of many other synthetic compounds.

Among others, chamigrene (+)-elatol has various pharmacological properties, exhibiting acaricidal and repellent activity, larvicidal activity, antimicrobial activity against human pathogenic bacteria and fungi, antiproliferative activity, anti-leishmania activity, anti-trypanocidal activity and antiviral activity.

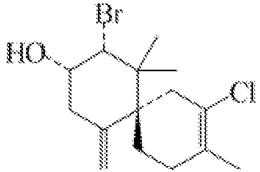

The challenge of the technique is to find new active ingredients to develop effective drugs for treating PAM caused by *Naegleria fowleri.* The present invention proposes chamigrene sesquiterpene compounds as active ingredients.

### Description of the invention

The present invention relates to the use of a chamigrene sesquiterpene compound for treating or preventing a *Naegleria fowleri* infection in a subject, preferably primary amebic meningoencephalitis (PAM).

In a preferred aspect, said chamigrene sesquiterpene is selected from (+)-elatol, (-)-elatol and (-)-rogiolol, even more preferably (+)-elatol.

In another preferred aspect, said chamigrene compound is selected from the group consisting of β-chamigrenes, nor-chamigrenes, chamigrenes with oxygenation capacity at C-7, or chamigrenes featuring a 5,10-epoxide ring or a carbocycle with prior migration of a methyl from C-11 to C-10.

Infections with *N. fowleri* have been reported both in humans and in animals including carnivores, horses, ruminants and some wild animals. Therefore, one aspect of the invention is that the infected subject is an animal and a more preferable aspect is that said subject is a human.

The chamigrene compounds show very low IC₅₀ cytotoxicity values compared to *N. fowleri* and, specifically, the (+)-elatol equivalent to that of amphotericin B; therefore, it is a possible alternative in the treatment of an infection by the microbe. Although amphotericin B shows low IC₅₀ values compared to mammalian cells, which has recommended its use, it is not really a valid reference because of its high toxicity *in vivo.* It is for this reason that the present invention proposes the chamigrene compounds as effective active ingredients that can serve as a basis for developing drugs with therapeutic value against infection in a patient.

Therefore, another aspect of the present invention is a pharmaceutical composition comprising a chamigrene sesquiterpene compound together with pharmaceutically acceptable excipients, and optionally another active ingredient, preferably an antifungal. In another more preferable aspect, said composition is for intravenous or nasal administration.

Another aspect of the invention is a method comprising administering at least one additional composition that includes at least another agent or drug, more preferably an antifungal, even more preferably caspofungin, natamycin, miconazole, voriconazole, fluconazole, or derivatives or a combination thereof.

One aspect of the invention is a method for treating an infection caused by *N. fowleri* that comprises administering a chamigrene sesquiterpene to a patient in need thereof. Another aspect comprises combining said chamigrene sesquiterpene with a second formulation that comprises a second active agent with fungicidal activity in combination.

Another preferable aspect is a kit comprising a formulation of a chamigrene sesquiterpene compound, and optionally a second compound wherein said compound is a fungicide, and instructions for preparing the active agent and its use against an *N. fowleri infection.*

### Examples

### Example 1. Isolation of the chamigrene compounds

Isolation of chamigrene compounds from freeze-dried samples of the red macroalgae *Laurencia dendroidea* was were carried out.

Different extracts in methanol:ethyl acetate (1:1), chloroform:methanol (1:1) and dichloromethane were obtained from the freeze-dried samples, by ultrasound-assisted maceration at room temperature for 15 min, followed by filtration and concentration under vacuum using a rotary evaporator at 40°C. The resulting extracts were chromatographed on a Sephadex^{®} LH-20 molecular exclusion column using a mixture of hexane:dichloromethane:methanol (2:1:1) as an eluent or on a silica flash column eluted with mixtures of increasing polarity of n-hexane, dichloromethane, ethyl acetate and methanol, producing between seven and twelve fractions depending on the extract. Based on the ¹H Nuclear Magnetic Resonance (NMR) analysis, the sesquiterpene-enriched fractions were subsequently processed by open silica gel column chromatography, medium-pressure Lobar LiChroprep Si-60 (40-63 µm, MERCK^{®}) and/or high performance liquid chromatography (HPLC) with a semi-preparative silica column using *n-*hexane:ethyl acetate mixtures of increasing polarity suited to the nature of the sample. A total of 12 known chamigrenes were isolated: (+)-elatol; (-)-elatol; (+)-debromoelatol; (-)-10-bromochamigra-3,7(14)-dien-9-ol; (+)-obtusol; (-)-dendroidiol; (+)-obtusane; (-)-3,10-dibromo-4-chloro-alpha-chamigrene; (-)-cartilagineol; (-)-rogiolol; (+)-isoobtusol and nifedipine.

The chemical structures of the isolated compounds were established by ¹H and ¹³C NMR, correlation spectroscopy (COSY), heteronuclear multiple bond correlation (HMBC) and heteronuclear single quantum correlation (HSQC) on a Bruker^{®} Avance 600 MHz system using CDCl₃. Chemical shifts were given in ppm relative to the solvent signals (CDCl₃: δ_{H} 7.26, δ_{C} 77.16). Mass spectrometry data were acquired using a VG-Autospec Fisons system in electrospray ionization mode. The specific rotations [α]_{D} were determined using a PerkinElmer^{®} Polarimeter (model 241), under conditions at 20°C, sodium D-line λ = 589 nm and cell pitch length 1 dm. The compounds were identified by comparing their spectroscopic data with the available literature.

### Example 2. Cell cultures

Two strains of *Naegleria fowleri* obtained from the American Type Culture Collection (ATCC^{®} 30808^{™} and ATCC^{®} 30215^{™}) were used (LG Promochem, Barcelona, Spain). Both strains were grown in 2% (w/v) Bacto Casitone (Thermo Fisher Scientific, Madrid, Spain) supplemented with 10% (v/v) fetal bovine serum, 0.3 µg/ml penicillin G sodium salt and 0.5 mg/ml streptomycin sulphate (Sigma-Aldrich, Madrid, Spain) at 37°C.

A murine macrophage cell line J774A.1 (ATCC # TIB-67, LG Promochem, Barcelona, Spain) was used for the cytotoxicity assays. The cells were cultured in DMEM (Dulbecco's Modified Eagle's Medium) supplemented with 10% fetal bovine serum and 10 µg/ml gentamicin (Sigma-Aldrich, Madrid, Spain). The culture was maintained at 37°C in a 5% CO₂ atmosphere.

### Example 3. In vitro activity against Naegleria fowleri and cytotoxicity

All tested compounds were dissolved in dimethyl sulfoxide (DMSO). The *in vitro* activity of the selected compounds was assessed by a colorimetric assay based on the alamarBlue^{®} reagent. Amoebas were seeded in 96-well plates at a concentration of 2 × 10⁵ cells/ml (50 ml). Next, serial dilutions (in the same medium as the amoebas) of the compound were prepared and added (50 ml) to the wells containing the *Naegleria fowleri* cells. Lastly, 10% of the total volume of alamarBlue^{®} was added to the wells and incubated under gentle stirring at 37°C for 48 hours. After that time, the emitted fluorescence was read on an EnSpire^{®} Multimode Plate Reader (PerkinElmer, Spain) using a 570 nm emission wavelength and a 585 nm excitation wavelength (Rizo-Liendo A. et al. "In Vitro Activity of Statins against Naegleria fowleri."; Pathog, vol. 8, no. 3, p. 122 - 2019; doi: 10.3390/ pathogens8030122).

To assess cytotoxicity, an assay based on the alamarBlue^{®} reagent was carried out. Briefly, the murine macrophages (2 × 10⁵ cells/ml) were incubated with serial dilutions of the test compound that were performed in the same culture medium. Lastly, alamarBlue^{®} was added to each well and kept at 37°C and 5% CO₂ for 24 hours. After that time, the emitted fluorescence was read on an EnSpire^{®} Multimode Plate Reader (PerkinElmer, Spain) using a 570 nm emission wavelength and a 585 nm excitation wavelength (Arberas-Jiménez I. et al. "Laurinterol from Laurencia johnstonii eliminates Naegleria fowleri triggering PCD by inhibition of ATPases," Sci. Rep., vol. 10, no. 1, p. 1-13, 2020. doi: 10.1038/s41598-020-74729-y).

### Example 4. Evaluation of the induction of programmed cell death mechanisms in Naegleria fowleri

The presence of different metabolic events characteristic of the programmed cell death process was evaluated with the use of commercial kits. The strain ATCC^{®} 30808^{™} was used to carry out the assays. The cells (5 × 10⁵ cells/ml) were incubated with the inhibitory concentration 90 of the product for 24 hours at 37°C. All results obtained were compared with healthy cells as a negative control (Rizo-Liendo, A. et al. "The type 2 statins, cerivastatin, rosuvastatin and pitavastatin eliminate Naegleria fowleri at low concentrations and by induction of programmed cell death (PCD)"; Bioorg. Chem., vol. 110, p. 104784, 2021, doi: 10.1016/j.bioorg.2021.104784.)

### Example 5. In vitro activity against primary amebic meningoencephalitis of the chamigrene collection

The values of *in vitro* activity against *Naegleria fowleri* ATCC^{®} 30808^{™} from the collection of isolated compounds are represented in Table 1.

**Table 1. Values of inhibitory concentration 50 (IC₅₀) against the strain ATCC^{®} 30808^{™} of Naegleria fowleri. The results are expressed as mean ± standard deviation and in µg/ml.**

| Compounds | IC₅₀ |
|---|---|
| (+)-elatol | 0.36 ± 0.03 |
| (-)-elatol | 12.27 ± 2.68 |
| (-)-rogiolol | 8.18 ± 0.92 |
| (+)-debromoelatol | > 25 |
| (-)-10-bromochamigra-3,7(14)-dien-9-ol | > 25 |
| (+)-obtusol | > 25 |
| (-)-dendroidiol | > 25 |
| (+)-obtusane | > 25 |
| (-)-3,10-dibromo-4-chloro-alpha-chamigrene | > 25 |
| (-)-cartilagineol | > 25 |
| isoobtusol + cartilagineol | > 25 |
| nifedipine + obtusane | > 25 |

The studies on activity with the available collection of compounds have identified that chamigrenes, with emphasis on the (+)-elatol compound, show excellent results for treating N. *fowleri.*

### Example 6. Comparison of the in vitro activity of marine chamigrenes with reference drugs against primary amebic meningoencephalitis

The activity and cytotoxicity of (+)-elatol, (-)-elatol and (-)-rogiolol as marine chamigrenes were evaluated against *Naegleria fowleri in vitro* compared to other similar compounds such as lauriterol and debromolauriterol, with reference to amphotericin B and miltefosine.

**Table 2. Values of inhibitory concentration 50 (IC₅₀) against the strains ATCC^{®} 30808^{™} and ATCC^{®} 30215^{™} of Naegleria fowleri and cytotoxic concentration 50 (CC₅₀) against murine macrophages J774A.1. The results are expressed as mean ± standard deviation and in µg/ml. SI= selectivity index.**

| Compound | ATCC^{®} 30808^{™} | | ATCC^{®} 30215^{™} | | CC₅₀ |
|---|---|---|---|---|---|
| | IC₅₀ | SI | IC₅₀ | SI | |
| (+)-elatol | 0.36 ± 0.03 | 57.02 | 0.38 ± 0.06 | 54.03 | 20.53 ± 4.33 |
| (-)-elatol | 12.27 ± 2.68 | 4.39 | - | - | 53.91 ± 4.28 |
| (-)-rogiolol | 8.18 ± 0.92 | 5.57 | - | - | 45.56 ± 7.80 |
| lauriterol | 3.96 ± 0.76 | 5.97 | 3.45 ± 0.63 | 6.85 | 23.65 ± 2.30 |
| debromolauriterol | 5.54 ± 1.19 | 2.74 | 14.13 ± 2.37 | 1.07 | 15.17 ± 2.80 |
| Amphotericin B | 0.11 ± 0.03 | > 1818.18 | 0.15 ± 0.02 | > 1333.33 | > 200 |
| Miltefosine | 15.79 ± 1.72 | 3.30 | 33.24 ± 2.95 | 1.56 | 52.12 ± 3.61 |

The activity values of the active chamigrenes, and in particular (+)-elatol, are shown to be comparable to the reference drug amphotericin B. Furthermore, all are much more active than the reference compound Miltefosine.

## Claims

1. Use of the (+)-elatol compound for preparing a medicine for treating or preventing a *Naegleria fowleri* infection in a subject.

2. The use according to claim 1, **characterized in that** said infection is primary amebic meningoencephalitis (PAM).

3. The use according to any of claims 1 to 2, **characterized in that** said subject is a human.

4. The use according to any of claims 1 to 2, **characterized in that** said subject is an animal.

5. An (+)-elatol compound for use in the treatment of the disease caused by *Naegleria fowleri.*

6. A pharmaceutical composition **characterized in that** it comprises a chamigrene sesquiterpene compound and a pharmaceutically acceptable excipient for use in the treatment of diseases caused by *Naegleria fowleri.*

7. The pharmaceutical composition according to claim 6, **characterized in that** it comprises an additional active ingredient.

8. The pharmaceutical composition according to claim 7, **characterized in that** said additional active ingredient is an antifungal.

9. The composition according to any of claims 6 to 8, **characterized in that** it is a composition for intravenous or nasal administration.

10. A kit comprising a formulation of a chamigrene sesquiterpene compound and optionally an additional compound wherein said compound is a fungicide, and instructions for preparing the active agent for use in the treatment of diseases caused by *Naegleria fowleri.*
